# EUROPEAN PATENT APPLICATION

(11) **EP 4 582 074 A2**
(43) Date of publication of application: **09.07.2025**
(21) Application number: 25177903.9
(22) Date of filing: 15.06.2018
(51) Int. Cl.: A61K 8/11

(54) **ABSORBENT ARTICLE**

(62) Divisional of application: 18922870.3
(71) Applicant: Essity Hygiene and Health Aktiebolag, 405 03 Göteborg (SE)
(72) Inventor: PALMQVIST, Lisa, 405 03 GÖTEBORG (SE); KNÖS, Anna, 405 03 GÖTEBORG (SE); ALM, Leif, 443 33 LERUM (SE); EKNESTEDT, Claes, 441 95 ALINGSÅS (SE)
(74) Representative: Hoffmann Eitle

(57) **Abstract**

An absorbent article comprising at least a topsheet layer and a backsheet layer, each layer having a first and a second surface, at least one of the layers has a composition comprising a skin beneficial agent printed on the first surface and an ink composition printed on the second surface, wherein the compositions on the first and the second surfaces respectively may be interchanged.

## Description

### TECHNICAL FIELD

The present disclosure pertains to an absorbent article, such as a sanitary napkin, a panty liner, an incontinence pad, an incontinence diaper, a belted diaper, or a baby diaper comprising a skin beneficial agent and a method for producing the same.

### BACKGROUND

Absorbent articles for personal hygiene are designed to absorb and contain body exudates. The absorbent articles normally comprise a topsheet layer facing the skin of the user, a backsheet layer facing the garment of the user and optionally an absorbent core located between these layers.

Absorbent articles may contain additives to provide certain advantages for the user. US20150282999A1 discloses an absorbent article with a chemical treatment in the form of a skin care composition on the surface of the topsheet.

### SUMMARY

The present disclosure is based on the insight how an absorbent article may be adopted for specific user requirement to thereby optimize the performance of the article for the user.

The present invention relates to an absorbent article according to claim 1.

Thus, the absorbent article comprises at least a topsheet layer and a backsheet layer, each layer having a first and a second surface, wherein at least one of the layers has a composition comprising a skin beneficial agent printed on the first surface and an ink composition printed on the second surface, and wherein the compositions on the first and the second surfaces respectively may be interchanged.

The skin beneficial agent may be enclosed in microcapsules.

The first surface of the layer may be the skin facing side and the second surface may be the garment facing side of the layer in the absorbent article.

The layer may comprise a zone of skin beneficial agent. The layer may comprise a zone of ink composition. A zone may have a boundary enclosing an area of at least 1 mm².

The zone of skin beneficial agent may have a non-linear boundary in at least the transversal direction of the article. The boundary of the zone may end at least 1 mm from the transversal edges of the layer. The boundary of the zone may end at least 1 mm from the longitudinal edges of the layer.

The ink composition may form an artwork on the surface of the layer.

The skin beneficial agent and the ink may be printed in different patterns. The skin beneficial agent and the ink may be printed in the same pattern.

The application pattern of the skin beneficial agent on the first surface and the application pattern of the ink on the second surface may not be synchronized in relation to each other.

The application pattern of the ink composition on the first surface and the application pattern of the skin beneficial agent on the second surface may be synchronized in relation to each other.

The method entails printing a material layer for an absorbent article, the material layer having a first and a second surface, comprising: a) conveying a material layer on a transporting means; b) printing a composition comprising a skin beneficial agent by means of a flexoprinter on the first surface of the material layer; c) printing an ink composition by means of a flexoprinter on the second surface of the material layer; d) wherein the application of the compositions in step b and c respectively may be interchanged.

The method may include a step preceding step c, wherein the material layer is conveyed to a web turning module turning the layer upside down. A drying step may succeed method step b.

The material layer may be printed by means of a first and a second flexoprinter printing stations located on a central impression cylinder.

A zone of skin beneficial agent is printed on the layer. The zone of skin beneficial agent may have a non-linear boundary in at least the transversal direction of the article.

An artwork may be printed by means of the ink composition.

The skin beneficial agent and the ink may be printed in different patterns. The skin beneficial agent and the ink may be printed in the same pattern.

The application pattern of the ink on the first surface and the application pattern of the skin beneficial agent on the second surface may or may not be synchronized in relation to each other.

The material layer may be a topsheet material layer or an acquisition material layer located between the topsheet and a core layer.

### BRIEF DESCRIPTION OF THE FIGURES

- Figure 1A: is a top view of an incontinence pad.
- Figure 1B: is a cross-sectional view of the pad in Figure 1A.
- Figure 2: is a top view of a topsheet of a pad having skin beneficial agent on its first surface.
- Figure 3: is a top view of a topsheet of a pad having ink print on its second surface.

### DETAILED DESCRIPTION

An absorbent article herein may be a sanitary napkin, a panty liner, an incontinence pad, an incontinence diaper, a belted diaper, or a baby diaper of open or closed type.

At least one of the layers of the absorbent article has a composition comprising a skin beneficial agent printed on the first surface and an ink composition printed on the second surface, wherein the compositions on the first and the second surfaces respectively may be interchanged. The skin beneficial agent may be enclosed in microcapsules. The skin beneficial agent may be applied in a zone. The ink composition may be applied in a zone. The first surface may be the skin facing surface allowing the user to come in close contact with the skin beneficial agent. The second surface may be the garment facing surface containing the ink and being located further away from the skin of the user.

A zone is an area which may be the same on every product and thus may be synchronized to the shape of the article. A zone may thus be distinguished from a continuous string, sheet or line in any pattern. Zones may be functional zones on the product in which the skin beneficial agent is precisely located through printing to give optimal performance of the additive on the product i.e. where the substance will be most effective e.g. in the most beneficial part of the product.

The zone of skin beneficial agent may be discrete with boundaries starting and ending within the edge borders of the layer. The boundary of the zone may end at least 1 mm from the transversal edges of the layer. The boundary of the zone may end at least 1 mm from the longitudinal edges of the layer.

There may be one, two or multiple zones. A first and second zone may be at least partly overlapping. A first zone may form a micropattern within a second zone.

A zone may have a boundary enclosing an area of at least 1 mm², or at least 10 mm², or at least 50 mm².

The skin beneficial agent may be applied on 1-100% of a surface area of a layer of the article, such as 1-90%, such as more than 1% and less than 90%, or more than 1% and less than 80%.

The zone of skin beneficial agent may have a rounded shape. The rounded shape of the zone may include a circular shape, an elliptic shape, a rectangular shape and a square shape with rounded corners. At least the first zone may have its center point in the crotch portion of the article.

The size of the microcapsules may be at least 1 µm, or at least 3 µm, or at least 10 µm and may be below 100 µm, or below 70 µm, or below 30 µm. The size of the microcapsules may be 1-100 µm, or 1-70 µm, or 10-50 µm.

The concentration of microcapsules on the surface of the layer may be at least 0,01 g/m², or at least 0,01 g/m² or at least 0,1 g/m² or at least 0,5 g/m² and below 17 g/m², or below 10 g/m² or below 5 g/m². The concentration of microcapsules on the surface of the layer may be 0,01 - 17 g/m², or 0,01 - 10 g/m² or 0,01 - 5 g/m².

An artwork may be printed by means of the ink composition. The ink composition may be water based and comprise at least a binder. The ink composition may be a standard formulation known to the skilled man in the art. The ink composition may include colored pigments or dyes or be colorless. The ink may also include typical printing additives well known to those skilled in the art such as solvents, co-solvents and processing aids. Solvent may include among other alcohols, esters, aldehydes and water. Binders may be, but are not limited to, polymers, resins, emulsions and mixtures based on styrenes, acrylates, acetates, alkydes, polyurethanes, nitrocellulose or other cellulose derivatives, polyglycols, polyvinylbutyrates, polyvinyl alcohols, polyvinyl pyrrolidone and derivatives or mixtures thereof. Constituents such as dispersants, surfactants, wetting aids, defoamers, antifoaming agents, waxes, silicones, viscosity modifiers, pH regulators, anti-slip agents and preservatives may also be present in the ink formula together with one or more of an encapsulated beneficial additive. The binder in the ink composition ensures the hardening of the ink as well as keeping the ink including the microcapsules in place on the material.

The skin beneficial agent may be a commercial lotion or may be selected from almond oil, argan oil, sesame seed oil, jojoba oil, grapeseed oil, shea butter, olive oil, coconut oil, avocado oil, limonene, linalool, geraniol, citral, coumarin, hibiscus, *Lavendula augustifolia,* calendula, chamomile, peppermint, sandalwood, peach, mango, apricot, sea buckthorn, coffee, vanilla, chocolate, menthol, xylitol. The beneficial agent may also be selected from carbamid, glycerin, dimethicone, tocopheryl (vitamin E), ascorbic acid (vitamin C), allantoin, thymol, salicylates. The skin beneficial agent may be enclosed in microcapsules.

The skin beneficial agent may be a phase change material, which may be enclosed in microcapsules. PCM material for the microcapsules may be a nonvolatile organic temperature regulating agent such as paraffin wax mixtures or polymers such as polyethylene glycols, fatty acids or ester derivatives of these (such as caprylic, capric, lauric and tridecylic acid and eutectic mixtures of these with palmitic, myristic or stearic acid) as well as polyalcohols, derivatives thereof and polyethylenes, or they may be an inorganic such as salt hydrates e.g. hydrated calcium and magnesium chlorides or hydrated carbonates. Examples of commercial producers of micro encapsulated PCMs suitable for body wear applications or energy storage applications in the comfort temperature interval for humans are Devan Chemicals (BE), Microtek Laboratories (US), Climator (SE) and MicroCaps (SLO).

The skin beneficial agent is applied by printing on the absorbent article. By printing we herein mean a precise application of a fluid to a substrate. By precise we mean that the medium will be placed in designated zones on the substrate, rather than in a poorly controlled fashion such as when using a spraying, coating or extrusion technique. The print is of a contact type in the form of flexoprint and may be applied in-line and synchronized, allowing for an exact placement on the layer.

The steps of in-line synchronized printing may be incorporated as steps in a process of manufacturing absorbent articles, or the material layers may be in-line synchronized printed before the assembly of the product.

The method for printing a material layer for an absorbent article, the material layer having a first and a second surface, comprises:
a) conveying a material layer on a transporting means;
b) printing a composition comprising a skin beneficial agent, by means of a flexoprinter, on the first surface of the material layer;
c) printing an ink composition, by means of a flexoprinter on the second surface of the material layer; wherein the application of the compositions in step b and c respectively may be interchanged.

A step of conveying the material layer to a web turning module, wherein the material is turned upside down may be preceding method step c. The web turning module may perform a 180 degrees turnover of the material layer to prepare the layer for the next printing step.

The material layer may be printed by means of a first and a second flexoprinter printing station located on a central impression cylinder. The flexoprinter printing station may comprise an anilox roll and a roll with a laser ingraved sleeve or a mounting sleeve with and thin polymeric cliche.

After application of the skin beneficial agent and ink on the absorbent article it will dry almost instantaneously. However, a drying step may be added, such as blowing hot air on the surface.

The tension of the material layer on the transporting means may be 0.5-50 N, such as 0.5-30 N, such as 0.5-20 N, to obtain an acceptable printed layer.

The skin beneficial agent and the ink composition may be printed in the same or different patterns. The application pattern of the skin beneficial agent on the first surface and the application pattern of the ink on the second surface may or may not be synchronized in relation to each other.

The composition of skin beneficial agent may be applied in selected areas as desired, and in any desired pattern. The present method allows very accurate zones to be formed.

When arranged in the absorbent article, the top sheet has body facing surface and a garment facing surface. The skin beneficial agent may be applied to the skin facing surface. By applying agent on the skin facing surface the user obtains a direct access to the agents. The agent may also be applied to an intermediate layer of the article. Depending on the location of the skin beneficial agent various advantageous functional effects can be obtained. The ink may be printed on the garment facing side of the topsheet, i.e. the surface furthest away from the skin to minimize skin contact.

The absorbent article comprises at least a topsheet layer and a backsheet layer and optionally an acquisition layer and an absorbent layer arranged between the topsheet and the backsheet layers.

Each layer of the absorbent article has a garment facing surface and a body facing surface, and the skin beneficial agent may be applied to any of said surfaces. The skin beneficial agent may be added to an intermediate layer, such as an acquisition layer, located beneath a topsheet.

The absorbent article may comprise a body facing topsheet of a nonwoven, a film or a laminate thereof or a foam, and a back sheet of a liquid impervious polymeric film material or a laminate of a film and a nonwoven material, and an absorbent layer comprising pulp and/or superabsorbent material and/or a fibrous web. The acquisition layer may be a nonwoven highloft material.

The backsheet material may be breathable or non-breathable film or nonwoven and film laminate. The back sheet is facing away from the user during use, and is opposite to the body facing topsheet layer of the absorbent article. In case of an absorbent article in the form of a pad, a fastening means may be applied on the garment facing side of the back sheet, which may be covered by a release paper or single wrap.

The absorbent article may comprise a core of absorbent material. The absorbent core may comprise a first and a second absorbent layer. The absorbent layers may be homogeneous structures or may in themselves be layered structures such as absorbent laminates of the same or different materials. The absorbent layers may have uniform thickness or may vary in thickness in different parts of the layers. Similarly, the basis weight and composition may vary within the absorbent layers. By way of example, an absorbent layer may comprise a mixture of absorbent and/or non-absorbent fibers and superabsorbent material, wherein the ratio of superabsorbent material to fibers may vary in the layer. The first and second absorbent layers may have any suitable shape, such as an hourglass shape with widened end portions and a narrow portion in the crotch portion, or a rectangular shape.

The absorbent core may be made up of any suitable absorbent or fluid uptake material as known in the art, such as one or more layers of cellulose fluff pulp, foam, fibre waddings, etc. The absorbent core may contain fibers or particles of highly absorbent polymer material, commonly known as superabsorbents, which are materials having the ability to absorb and retain large quantities of fluid upon formation of a hydrogel. The superabsorbents may be mixed with cellulose fluff pulp and/or may be arranged in pockets or layers in the absorbent core. The fibres may be pulp fibres and the superabsorbent material may be polyacrylate-based particles. The absorbent core may further incorporate components for improving the properties of the absorbent core. Some examples of such components are binder fibers, fluid-dispersing materials, wetness indicators etc., as known in the art.

The activation of microcapsules containing skin beneficial agent may be performed by mechanical and/or heat activation upon contact. Not all microcapsules will be activated at the same time as some may be buried further down in the material and there will thus be a slow, continuous and beneficial activation during use of the article. A long-lasting effect can thus be achieved.

The application by print allows for a precise placement of a delicate printed zone in chosen areas on the article, compared with when the additive is applied for example as a constituent of the spin finish on a topsheet, in a so-called cocktail, which is commonly used by nonwoven suppliers. To further increase the benefits the print may be combined with precise in-line positioning (synchronization) of the print on any product. This enables the print to be placed in exact zones or areas, i.e. particular functional zones of the product. In this way the agent will be applied only in the printed zones, thus allowing for less amount and possibility for tailor made zones or areas.

Print including skin beneficial agent may be applied in different layers of the product. The topsheet, an intermediate layer, core or acquisition layer, or on a backsheet, glued part, wrap or release paper may be printed. More than one printed area, having the same or different printed additives, are possible on the same layer of the product and on different layers in the product. The printed beneficial zones may be placed within an absorbing area or outside of the absorbing area of an article.

The disclosure will now be described by way of example, referring to the drawings.

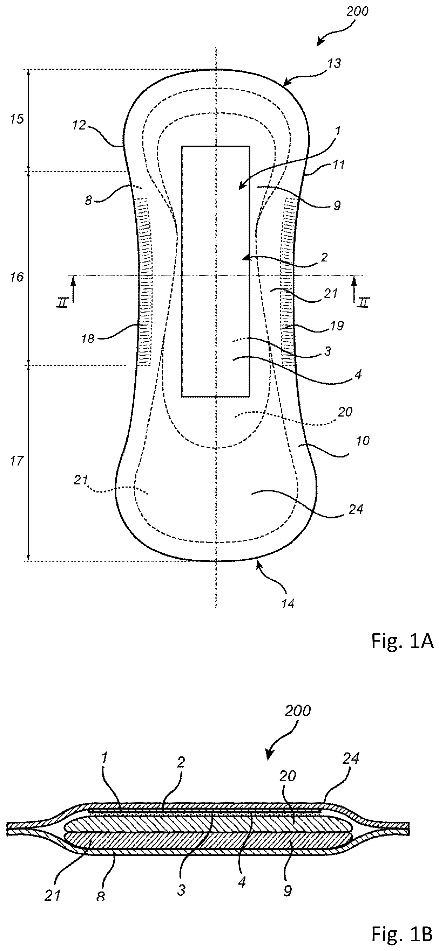

The absorbent article 200 shown in Fig. 1A and 1B is a urine incontinence protector in the form of a pad. The pad is seen from the side of the pad that is intended to be facing towards a wearer's body when being worn. The pad comprises a liquid permeable topsheet 24, a liquid impermeable backsheet 8, and an absorbent core 9 enclosed between the topsheet 24 and the backsheet 8, and an acquisition and distribution layer 1 arranged between the topsheet 24 and the absorbent core 9.

The topsheet 24 and the backsheet 8 of the pad extend together laterally outside of the absorbent core 9 along the whole circumference of the absorbent core 9 and is connected to each other in an edge joint 10 around the periphery of the absorbent core 9. The topsheet 24 comprises any material which is suitable for the purpose, i.e. soft and liquid pervious.

The backsheet 8 is fluid impermeable. However, backsheet materials that are only fluid repellant may be used particularly in instances where relatively small amounts of urine are expected to be taken up. Furthermore, the backsheet 8 may have an outer, garment-facing surface of a textile material such as nonwoven.

The absorbent core 9 may be made up of any suitable absorbent or fluid uptake material as known in the art, such as one or more layers of cellulose fluff pulp combined with fibers or particles of highly absorbent polymer material.

The pad in Fig. 1A has an elongate, generally rectangular shape when fully extended in all directions. Any suitable shape may be used for the absorbent product, such as hourglass shape, trapezoidal shape, triangular shape an oval shape, etc. The shape of the product may be symmetrical about a transverse center line through the product, or may be asymmetrical with end portions having differing shapes and/or differing sizes. The pad has two longitudinal side edges 11, 12 extending generally in the same direction as a longitudinal center line through the absorbent product. Front and rear end edges 13, 14 typically extend transversely to the longitudinal center line at the ends of the absorbent product. The rear end edge 14 is intended to be orientated rearwards during use of the absorbent article, and the front-end edge 13 is intended to be facing forwards towards the abdomen of the wearer. The pad has a longitudinal front portion 15, a longitudinal back portion 17 and a crotch portion 16 located intermediate the front and back portions 15, 17. The crotch portion 16 is a portion which is intended to be placed against the crotch of a wearer during use of the product and to constitute the main acquisition area for body fluid that reaches the pad. The pad may further include fastening means for fastening of the absorbent product inside a supporting pant garment, such as a pair of underpants.

Elastic elements 18, 19 may be arranged along the side edges laterally outside the absorbent core 8. The elastic elements 18, 19 may be bands of elastic material. The elastic elements 18, 19 are optional components of the absorbent product and may be omitted.

The acquisition and distribution material 1 in Fig. 1A is situated above the absorbent core 9 and beneath and in direct contact with the topsheet 24 and may be a nonwoven high loft material or a perforated material such as a SMS material.

The absorbent core 9 in Fig. 1A has a first absorbent layer 20 and a second absorbent layer 21. The second absorbent layer 21 is placed below the first absorbent layer 20. The first absorbent layer 20 is smaller than the second absorbent layer 21. The second absorbent layer 21 extends further forward and rearward in the absorbent product than the first absorbent layer 20. However, the absorbent core may also comprise only one single layer or may comprise one or more further absorbent layers. The size of the different layers may also vary, and the absorbent core 9 described in figure 1A and 1B is only one illustration of an absorbent core.

In Fig. 1B a cross-sectional view of the absorbent pad of Figure 1A is shown, along the line II-II. The pad has a liquid permeable top sheet 24, a liquid impermeable back sheet 8, and an absorbent core 9 enclosed between the top sheet 24 and the back sheet 8 and an acquisition and distribution material 1 is located between the topsheet 24 and the absorbent core 9.

### Example 1

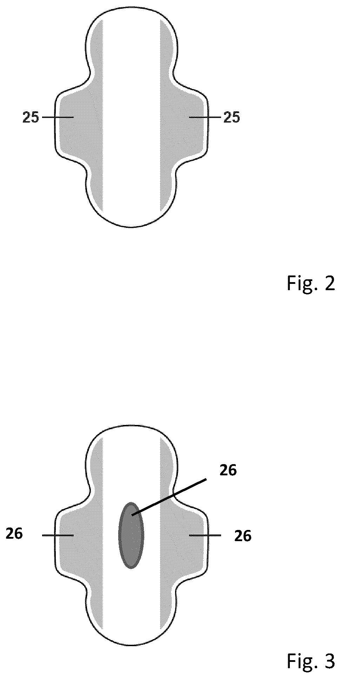

A topsheet material for a feminine pad was printed with zones with a flexographic printer according to area 25 in Fig. 2, wherein one set denoted 1a) had microencapsulated PCM printed on the body facing surface of the topsheet on the left and right-hand side of the center line in the longitudinal direction. The outer side of the zone follows the outer contour of the product but also the area being folded around the underwear to create a good comfort against the skin. A small portion, 3 mm wide, of the outermost area on the pad was not covered by the PCM. The PCM material was MPCM32 wet cake, Microtek laboratories (temp interval 30-35°C) and the concentration was 1.8 g/m². The garment facing side of the topsheet material was printed with an ink printed artwork 26, wherein the zones 26 on the left and right-hand side of the center line in the longitudinal direction was synchronized with the PCM zones 25 on the body facing surface. The water based ink used was Kappaflex purple 98-2086 from Kapp-Chemie, Germany.

A second set of topsheet material denoted 1b) was printed with microencapsulated additive Salpshere #8953 with seaweed and vitamins A, C and E from Salvona Technologies US, and with the concentration 0,15 g/m², in the same pattern on the body facing surface of the topsheet as above for 1a). The garment facing side of the topsheet was printed with an ink printed artwork using the same ink and pattern as above in 1b).

The present application includes the following items 1-28:
1. An absorbent article comprising at least a topsheet layer and a backsheet layer, each layer having a first and a second surface, at least one of the layers has a composition comprising a skin beneficial agent printed on the first surface and an ink composition printed on the second surface, wherein the compositions on the first and the second surfaces respectively may be interchanged.
2. Absorbent article according to item 1, wherein the skin beneficial agent is enclosed in microcapsules.
3. Absorbent article according to item 1 or 2, wherein the first surface is the skin facing surface and the second surface is the garment facing surface of the article.
4. Absorbent article according to any one of the items 1-3, wherein the layer comprises a zone of skin beneficial agent.
5. Absorbent article according to any one of the items 1-4, wherein the layer comprises a zone of ink composition.
6. Absorbent article according to item 5, wherein the zone of skin beneficial agent has a non-linear boundary in at least the transversal direction of the article.
7. Absorbent article according to any one of the items 4-6, wherein the boundary of the zone ends at least 1 mm from the transversal edges of the layer.
8. Absorbent article according to any one of items 4-7, wherein the boundary of the zone ends at least 1 mm from the longitudinal edges of the layer.
9. Absorbent article according to any one of items 4-8, wherein the zone has a boundary enclosing an area of at least 1 mm².
10. Absorbent article according to any one of the preceding items, wherein the ink forms an artwork on the surface of the layer.
11. Absorbent article according to any one of the preceding items, wherein the skin beneficial agent and the ink are printed in different patterns.
12. Absorbent article according to any one of the preceding items, wherein the skin beneficial agent and the ink are printed in the same pattern.
13. Absorbent article according to any one of the preceding items, wherein the application pattern of the skin beneficial agent on the first surface and the application pattern of the ink on the second surface are not synchronized in relation to each other.
14. Absorbent article according to any one of the preceding items, wherein the application pattern of the skin beneficial agent on the first surface and the application pattern of the ink on the second surface are synchronized in relation to each other.
15. Method for printing a material layer for an absorbent article, the material layer having a first and a second surface, comprising:
   a) conveying a material layer on a transporting means;
   b) printing a composition comprising a skin beneficial agent, by means of a flexoprinter, on the first surface of the material layer;
   c) printing an ink composition, by means of a flexoprinter, on the second surface of the material layer;
   wherein the application of the compositions in steps b and c respectively may be interchanged.
16. Method according to item 15, wherein the material layer is printed by means of a first and a second flexoprinter printing stations located on a central impression cylinder.
17. Method according to items 15 or 16, wherein a step of conveying the material layer to a web turning module, wherein the material is turned upside down, is preceding method step c.
18. Method according to any one of items 15-17, wherein a drying step is succeeding method step b.
19. Absorbent article according to any one of the items 15-18, wherein a zone of skin beneficial agent is printed on the layer.
20. Absorbent article according to any one of the items 15-19, wherein a zone of ink composition is printed on the layer.
21. Absorbent article according to items 19 or 20, wherein the zone has a non-linear boundary in at least the transversal direction of the article.
22. Method according to any one of items 15-21, wherein an artwork is printed by means of the ink composition.
23. Method according to any one of the items 15-22, wherein the skin beneficial agent is enclosed in microcapsules.
24. Method according to any one of the items 15-23, wherein the skin beneficial agent and the ink composition are printed in different patterns.
25. Method according to any one of the items 15-24, wherein the skin beneficial agent and the ink composition are printed in same pattern.
26. Method according to any one of the items 15-25, wherein the application pattern of the skin beneficial agent on the first surface and the application pattern of the ink on the second surface are not synchronized in relation to each other.
27. Method according to any one of the items 15-26, wherein the application pattern of the skin beneficial agent on the first surface and the application pattern of the ink on the second surface are synchronized in relation to each other.
28. Method according to any one of the items 15-27, wherein the material layer is a topsheet material layer or an acquisition material layer located between the topsheet and a core layer.

## Claims

1. An absorbent article comprising at least a topsheet layer and a backsheet layer, each layer having a first and a second surface, at least one of the layers has a composition comprising a skin beneficial agent printed on the first surface being the skin facing surface and an ink composition printed on the second surface being the garment facing surface.

2. Absorbent article according to claim 1, wherein the skin beneficial agent is enclosed in microcapsules.

3. Absorbent article according to claim 1 or 2, wherein the layer comprises a zone of skin beneficial agent.

4. Absorbent article according to any one of the claim 1-3, wherein the layer comprises a zone of ink composition.

5. Absorbent article according to claim 4, wherein the zone of skin beneficial agent has a non-linear boundary in at least the transversal direction of the article.

6. Absorbent article according to any one of the claims 3-5, wherein the boundary of the zone ends at least 1 mm from the transversal edges of the layer.

7. Absorbent article according to any one of claims 3-6, wherein the boundary of the zone ends at least 1 mm from the longitudinal edges of the layer.

8. Absorbent article according to any one of claims 3-7, wherein the zone has a boundary enclosing an area of at least 1 mm².

9. Absorbent article according to any one of the preceding claims, wherein the ink forms an artwork on the surface of the layer.

10. Absorbent article according to any one of the preceding claims, wherein the skin beneficial agent and the ink are printed in different patterns.

11. Absorbent article according to any one of the preceding claims, wherein the skin beneficial agent and the ink are printed in the same pattern.

12. Absorbent article according to any one of the preceding claims, wherein the application pattern of the skin beneficial agent on the first surface and the application pattern of the ink on the second surface are not synchronized in relation to each other.

13. Absorbent article according to any one of the preceding claims, wherein the application pattern of the skin beneficial agent on the first surface and the application pattern of the ink on the second surface are synchronized in relation to each other.
